# EUROPEAN PATENT APPLICATION

(11) **EP 3 043 169 A1**
(43) Date of publication of application: **13.07.2016**
(21) Application number: 15203259.5
(22) Date of filing: 31.12.2015
(51) Int. Cl.: G01N 21/85, A61M 1/36, A61M 5/36, G01N 33/49

(54) **NON-INVASIVE IDENTIFICATION OF SOLUTIONS**

(30) Priority: 06.01.2015 US 201514590299
(71) Applicant: Fenwal, Inc., Lake Zurich, IL 60047 (US)
(72) Inventor: Sandford, Craig, Lake Zurich, IL 60047 (US)
(74) Representative: Jenkinson, Kay Elizabeth

(57) **Abstract**

A fluid identification assembly (200) is provided to determine the presence or absence of multiple components within a fluid (210) in a conduit (202). The assembly includes a light source (206) and a light detector (208) positioned to receive a portion of light (L) from the light source (206) reflected by the conduit (202) and fluid (210). A dual-band bandpass filter may be associated with the light detector to allow multiple wavelengths of light to be monitored, thereby monitoring the composition of the fluid based on the amount of light of each wavelength absorbed by the fluid. Rather than a dual-band bandpass filter, a second light detector (212) may be provided, along with a beam splitter (214) to separate light from a single source (206) for receipt of light of different wavelengths (L1, L2) by the light detectors (208, 212). The dual-band bandpass filter may also be replaced by a second light source and detector pair for analyzing fluid using multiple wavelengths of light.

## Description

### Cross-Reference To Related Applications

This application claims priority from U.S. patent application no. 14/590,299.

### Field of the Disclosure

The disclosure relates to detection of fluid properties. More particularly, the disclosure relates to systems and methods for optically detecting or monitoring multiple components of a fluid.

### Description of Related Art

Various blood processing systems now make it possible to collect particular blood constituents, instead of whole blood, from a blood source. Typically, in such systems, whole blood is drawn from a blood source, the particular blood component or constituent is separated, removed, and collected, and the remaining blood constituents are returned to the blood source. Removing only particular constituents is particularly advantageous when the blood source is a human donor, because potentially less time is needed for the donor's body to return to pre-donation levels, and donations can be made at more frequent intervals than when whole blood is collected. This increases the overall supply of blood constituents, such as plasma and platelets, made available for transfer and/or therapeutic treatment.

Whole blood may be separated into its constituents through any of a number of automated procedures, such as centrifugation, membrane separation, and others. Centrifugation, for example, requires that the whole blood be passed through a centrifuge after it is withdrawn from, and before it is returned to, the blood source. To reduce contamination and possible infection (if the blood source is a human donor or patient), the blood is preferably processed within a sealed, sterile fluid flow system during the centrifugation process. Typical blood processing systems include a disposable, sealed, and sterile flow circuit, including a centrifuge chamber portion, that is mounted in cooperation on a durable, reusable assembly containing the hardware (centrifuge, drive system, pumps, valve actuators, programmable controller, and the like) that rotates a centrifuge chamber and controls the flow through the fluid circuit.

In some cases, the disposable flow circuit is configured to allow attachment of additional components, such as bags containing fluids used during the procedure. These fluids may be used for different purposes, such as anticoagulant fluid for preventing the coagulation of blood outside of the body, storage fluid to be added to separated blood components for long-term storage, and replacement fluid to be infused into a patient or donor to replace blood components that are being stored for later use. Some fluids that are used for one purpose may be harmful if used for a different purpose. For example, sodium citrate is commonly used as an anticoagulant, but the excess infusion of sodium citrate into a patient or donor may be potentially harmful by overly increasing the citrate concentration of the blood. Accordingly, care must be taken to ensure that the proper fluid containers are connected to the various ports or access points of a disposable flow circuit.

In addition to identification of citrate in a replacement fluid conduit, there are situations where it would be advantageous to identify other types of fluids, including identifying more than one component of a fluid to distinguish between fluids having a common fluid component. For example, it may be advantageous to not only identify a citrate-containing fluid, but also to identify at least a second component of the fluid to distinguish among various citrate-containing fluids.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a fluid identification assembly is provided for use in combination with a fluid container. The fluid identification assembly includes a light source, a light detector, and a dual-band bandpass filter. The light source is configured to direct a light substantially transmitted by the fluid container to the fluid container for reflection by the fluid container and by a fluid present in the fluid container, with the light detector being positioned to receive at least a portion of the reflected light. The dual-band bandpass filter is positioned such that at least a portion of the light passes through the dual-band bandpass filter prior to being received by the light detector. The dual-band bandpass filter is configured to allow light having a first wavelength or a first range of wavelengths and a second wavelength or a second range of wavelengths to reach the light detector and to substantially prevent other wavelengths of light from reaching the light detector. The light detector is configured to, after receiving the reflected light passing through the dual-band bandpass filter, generate a signal indicative of the identity of the fluid present in the fluid container based, at least in part, on the amount of light received from the light source.

In another aspect, a fluid identification assembly is provided for use in combination with a fluid container. The fluid identification assembly includes a light source, a beam splitter, and first and second light detectors. The light source is configured to direct a light substantially transmitted by the fluid container to the fluid container for reflection by the fluid container and by a fluid present in the fluid container. The beam splitter is positioned such that at least a portion of the light reflected by the fluid container and by the fluid present in the fluid container is intercepted by the beam splitter and separated into a first separated light beam and a second separated light beam. The first light detector is positioned to receive at least a portion of the first separated light beam, while the second light detector is positioned to receive at least a portion of the second separated light beam. The first light detector is configured to, after receiving at least a portion of the first separated light beam, generate a first signal indicative of the presence or absence of a first fluid component in the fluid present in the fluid container based, at least in part, on the amount of light received from the light source. The second light detector is configured to, after receiving at least a portion of the second separated light beam, generate a second signal indicative of the presence or absence of a second fluid component in the fluid present in the fluid container based, at least in part, on the amount of light received from the light source.

In yet another aspect, a fluid identification assembly is provided for use in combination with a fluid container. The fluid identification assembly includes first and second modules, with the first module having a first light source and a first light detector and the second module having a second light source and a second light detector. The first light source is configured to direct a first light substantially transmitted by the fluid container to the fluid container for reflection by the fluid container and by a fluid present in the fluid container, with the first light detector being positioned to receive at least a portion of the reflected first light. The second light source is configured to direct a second light substantially transmitted by the fluid container to the fluid container for reflection by the fluid container and by a fluid present in the fluid container, with the second light detector being positioned to receive at least a portion of the reflected second light. The first light detector is configured to, after receiving at least a portion of the reflected first light, generate a first signal indicative of the presence or absence of a first fluid component in the fluid present in the fluid container based, at least in part, on the amount of light received from the first light source. The second light detector is configured to, after receiving at least a portion of the reflected second light, generate a second signal indicative of the presence or absence of a second fluid component in the fluid present in the fluid container based, at least in part, on the amount of light received from the second light source.

In another aspect, a method is provided for identifying a fluid in a fluid container. The method includes directing a light substantially transmitted by a fluid container to the fluid container for reflection by the fluid container and by a fluid present in the fluid container. Reflected light having a first wavelength or a first range of wavelengths and a second wavelength or a second range of wavelengths is detected, and the identity of the fluid present in the fluid container is determined based, at least in part, on the amount of reflected light detected.

### Brief Description of the Drawings

Fig. 1 is a perspective view of an exemplary durable blood processing system that may be used in combination with citrate detectors according to the present disclosure;
Fig. 2 is a perspective view of a disposable set usable in association with the durable fluid processing system of Fig. 1;
Fig. 3 is a side elevational view of the disposable set of Fig. 2 mounted on the durable blood processing system of Fig. 1, which is partially broken away;
Fig. 4 is a side detail view of a centrifuge included in the durable blood processing system of Fig. 1, showing the centrifuge in combination with an umbilicus of the disposable set;
Fig. 5 is a diagrammatic view of a citrate detector that may be used in combination with the blood processing system and disposable set of Fig. 3;
Fig. 6 is a diagrammatic view of a fluid detector that may be used in combination with the citrate detector of Fig. 5;
Fig. 7 is a side elevational view of an alternative embodiment of a citrate detector according to an aspect of the present disclosure;
Fig. 8 is a top plan view of the citrate detector of Fig. 7;
Fig. 9 is a side elevational view of a fluid identification system according to an aspect of the present disclosure;
Fig. 10 is a top plan view of another alternative embodiment of a fluid identification system according to an aspect of the present disclosure; and
Fig. 11 is a side elevational view of an alternative embodiment of a fluid identification system according to an aspect of the present disclosure.

### Description of the Illustrated Embodiments

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

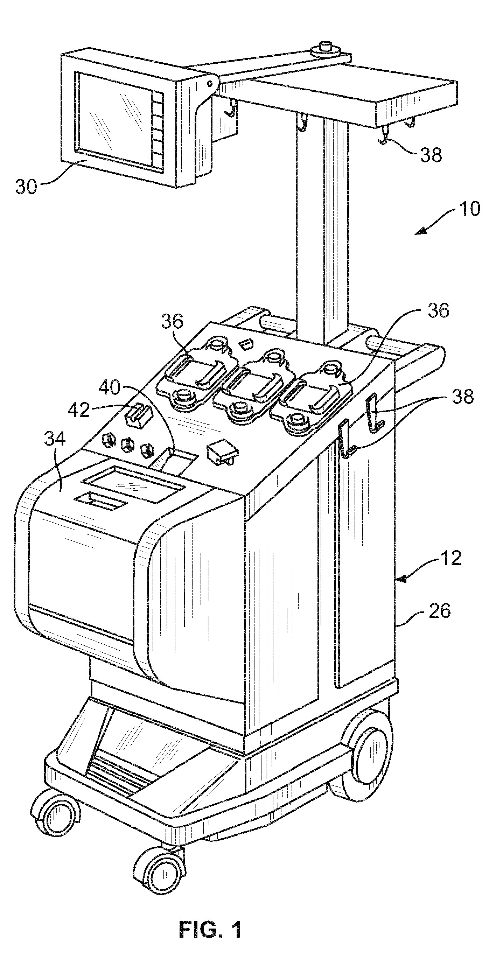

Fig. 1 shows a centrifugal blood processing system 10. The system is currently marketed as the AMICUS® separator by Fenwal, Inc. of Lake Zurich, Illinois. The system 10 can be used for processing various fluids, but is particularly well suited for processing whole blood, blood components, or other suspensions of biological cellular materials. The system 10 includes a centrifuge assembly 12 for separating a fluid into its constituent parts. A more detailed description of the centrifuge assembly 12 and the other elements of the system 10 can be found in U.S. Patent No. 5,996,634, which is incorporated by reference herein. While Fig. 1 illustrates a centrifugal blood processing system, it should be understood that the illustrated system is merely exemplary, and other blood processing systems (including filter- or membrane-based blood separators and other centrifugation or other separation systems) may be used in combination with citrate detectors according to the present disclosure.

When used for processing blood, a blood component, or any other body fluid, devices and methods according to the present disclosure may be used with any suitable fluid source. For example, the fluid source may be a living human or non-human animal whose bodily fluid is directly drawn into the device for processing. In other embodiments, the fluid to be processed does not come directly from a living human or non-human animal, but is instead provided directly from a non-living source, such as a container holding an amount of fresh or stored fluid (e.g., blood or a blood component that has been previously drawn from a living source and stored). In additional embodiments, there may be a plurality of fluid sources, which may all be living sources or non-living sources or a combination of living and non-living sources.

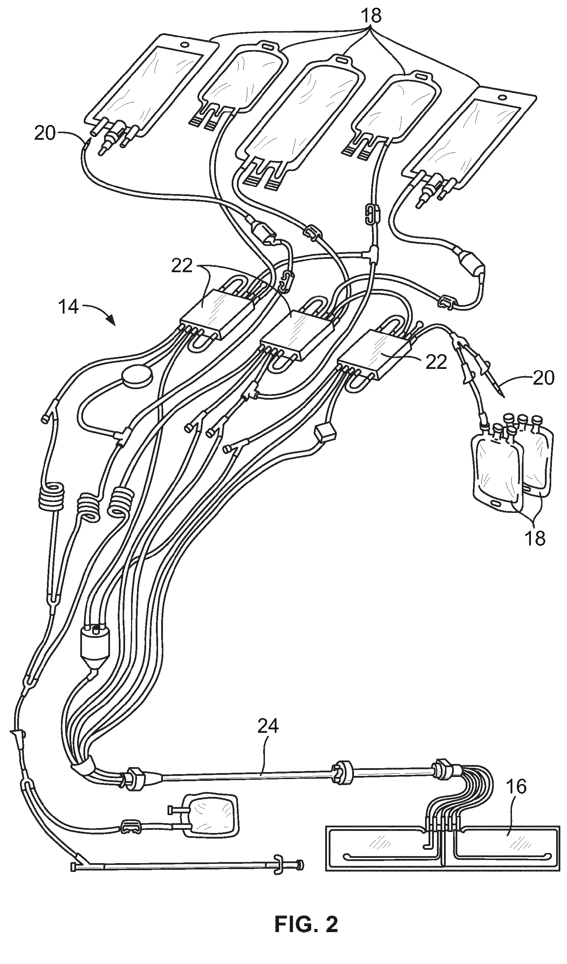

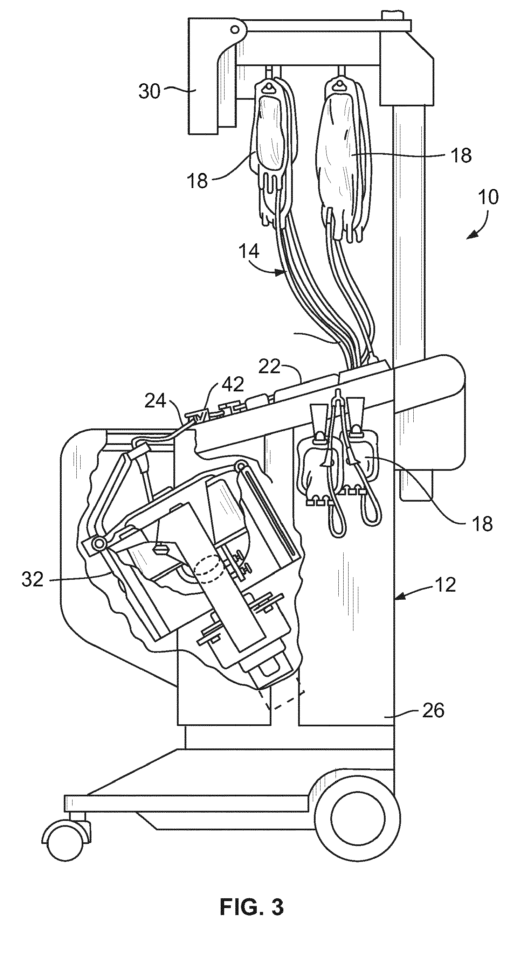

The durable blood processing system 10 is used in combination with a disposable processing set 14, an example of which is shown in Fig. 2. Fig. 3 shows the disposable set 14 mounted on the durable system 10. The disposable set 14 is a preferably single use, disposable item loaded on the system 10 at the time of use. After a fluid processing procedure has been completed, the operator preferably removes the disposable set 14 from the system 10 and discards it. It should be understood that the illustrated disposable set 14 of Fig. 2 is merely exemplary. The illustrated disposable set 14 is suitable for use with a system 10 as shown in Fig. 1, but those of ordinary skill in the art will appreciate that differently configured disposable sets are appropriate for use with different blood processing systems.

The disposable set 14 includes a processing chamber 16 (Fig. 2). In use, the centrifuge assembly 12 rotates the processing chamber 16 to centrifugally separate blood components. Whole blood is conveyed to the processing chamber 16, and separated blood components are conveyed from the processing chamber 16, through a plurality of flexible tubes that form part of a fluid circuit. The fluid circuit further includes a plurality of containers 18 that may be supported by elevated hangers located over the centrifuge assembly 12 (see Fig. 3) and that dispense and receive liquids during processing. At least some of the containers 18 are separate from the disposable set 14 and connected thereto by connectors 20. The nature of the connectors 20 may vary without departing from the scope of the present disclosure. For example, the connectors 20 may be cannulas configured to puncture the containers 18 to establish fluid communication between the fluid circuit and the containers 18. Other connectors and means for connecting separate containers to a disposable set 14, such as sterile connection devices, are known to those of ordinary skill in the art and may be employed without departing from the scope of the present disclosure. Preferably, the disposable set 14 is a pre-assembled closed system, with the connectors 20 allowing for sterile connection of the containers 18 to the fluid circuit.

Fluid flow through the fluid circuit 14 may be controlled in a variety of ways. In one embodiment, fluid flow is controlled via cassettes 22 with pre-formed fluid passageways, which may be selectively opened and closed pneumatically, hydraulically, or by movable actuators. The number of cassettes may vary, but in the illustrated embodiment, there are three cassettes 22, which operate in association with valve and pump stations on the centrifuge assembly 12 to direct liquid flow among multiple liquid sources and destinations during a blood processing procedure. Tubes connected to the processing chamber 16 lead to a flexible umbilicus 24, with additional tubes at the other end of the umbilicus 24 fluidly connecting the processing chamber 16 (via the umbilicus 24) to the remainder of the disposable set 14, including the containers 18 and the cassettes 22.

As illustrated, the centrifuge assembly 12 includes a wheeled cabinet 26 that can be easily rolled from place to place. A user-actuable processing controller 30 is provided which enables the operator to control various aspects of the blood processing procedure. A centrifuge rotor assembly 32 is provided behind a fold-open door 34 (Fig. 1) that can be pulled open at the front of the cabinet 26. A plurality of valve and pump stations 36 (Fig. 1) are provided on the top face of the cabinet for receiving and controlling the various cassettes 22. A plurality of hooks or hangers 38 are provided on the cabinet 26 for suspending the various containers 18.

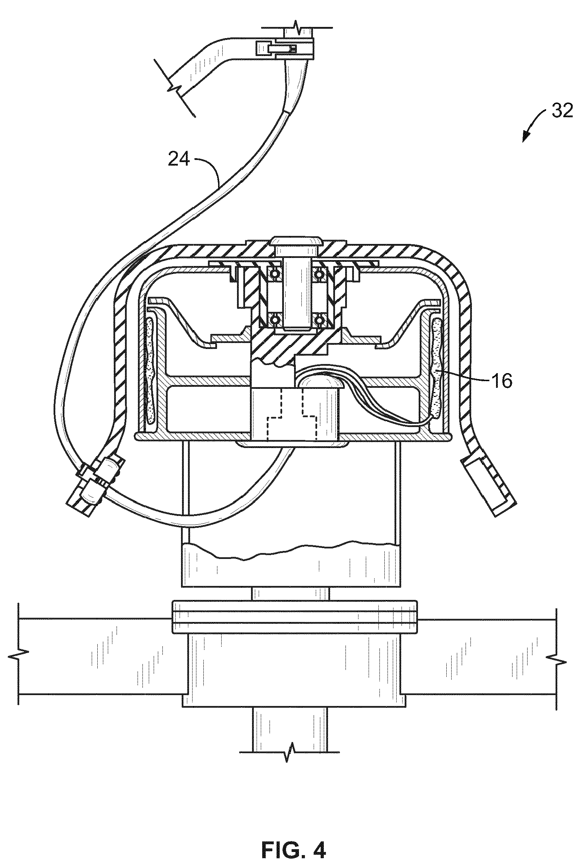

In use, the fold open door 34 is opened and the processing chamber 16 of the disposable set 14 is mounted in the centrifuge rotor assembly 32 (Fig. 4). The umbilicus 24 is threaded through the centrifuge rotor assembly 32 and out through an opening 40 in the upper panel of the cabinet 26 (Fig. 3). The cassettes 22 are snapped into respective ones of the valve and pump stations 36 and the containers 18 are hung from the appropriate hangers 38 (Fig. 3). After appropriate connections are made to the patient or donor using known intravenous techniques, the operator enters appropriate commands on the processing controller 30 to begin the desired processing procedure.

In an exemplary processing procedure, whole blood or a fluid containing one or more blood components is drawn into the disposable set 14 by a phlebotomy needle or other access device. To reduce clotting, anticoagulant is added to the fluid from one of the containers 18. The anticoagulated blood flows into the processing chamber 16, where it is separated into at least two components based on their relative densities (e.g., a relatively high density component, such as red blood cells, and a relatively low density component, such as platelet-rich plasma). One of the components (the high density component in one embodiment) may be pumped out of the processing chamber 16 and into one of the container 18, which serves as a storage container. The storage container may be provided with an amount of storage fluid or, alternatively, storage fluid may be added to the storage container from one of the other containers 18. The other component (the low density component in one embodiment) may be pumped out of the processing chamber 16 and returned to the blood source. Replacement fluid, such as saline, may be added to the fluid that is returned to the blood source to compensate for the blood volume deficit caused by storage of the selected blood component(s) in the storage container after separation. Other blood processing protocol may be carried out using the system 10 and disposable set 14 (e.g., procedures in which one of the separated components is further fractionated to form two or more sub-components), and it should be understood that the foregoing procedure is merely exemplary, rather than limiting.

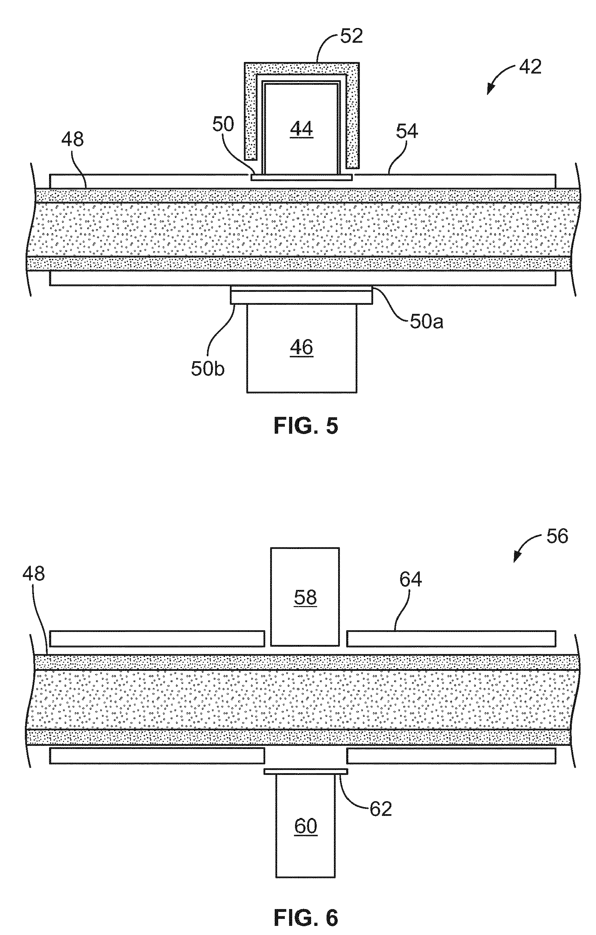

To better ensure that the separate containers 18 are properly connected to the remainder of the disposable set 14 and to protect a donor or patient from overexposure to citrate, which is a common component of commercial anticoagulants, the system 10 may be provided with a citrate detector 42 (Figs. 1 and 5). The citrate detector 42 includes a light source 44 (such as, but not limited to, one or more light-emitting diodes) and a light detector 46 (such as, but not limited to, a photodiode), with the light detector 46 being configured and oriented to receive light from the light source 44. The light source 44 and light detector 46 are spaced apart a sufficient distance to accommodate a conduit or length of tubing 48 therebetween. The conduit 48 is at least a portion or segment of the means by which replacement fluid is intended to flow from the appropriate container 18 into the disposable set 14. Fig. 1 illustrates the citrate detector 42 as being associated with the top face of the cabinet 26, but may be located anywhere else appropriate to accommodate the replacement fluid conduit 48.

The light source 44 emits a light having a wavelength that is absorbed at a higher level by citrate than by another fluid, e.g., replacement fluid (such as saline or another sodium chloride mixture) or by the conduit 48. Preferably, the light emitted by the light source 44 has a wavelength that is substantially transmitted through the replacement fluid and the conduit 48, with little to no absorption of the light. It has been found that ultraviolet light in the range of approximately 200 to 250 nm is absorbed by citrate or a citrate solution, but is substantially transmitted by or absorbed at a significantly lower level by sodium chloride solutions and by tubing manufactured from a variety of suitable materials (e.g., cyclic olefins, polyolefin, high density polyethylene, TPX® polymethylpentene, and fluorinated ethylene propylene).

In one embodiment, the conduit 48 through which light from the light source 44 is passed is a length of tubing (particularly tubing for the flow of a replacement fluid), but it should be understood that the present disclosure is not limited to tubing, but may include any other light-transmissive fluid flow conduit or structure or vessel through which a fluid, such as a replacement fluid, may pass. For example, it is within the scope of the present disclosure for replacement fluid flowing through flow conduit comprising a rigid, non-tubular optical chamber to be analyzed by light from a light source according to the foregoing description. In particular, it may be advantageous to provide a conduit or cuvette having relatively flat or planar surfaces through which the light from the light source 44 passes into and out of the conduit. By providing flat or planar surfaces instead of curved surfaces for the passage of light, the effect of adverse optical effects may be reduced, thereby improving the accuracy of the citrate detector 42.

Accordingly, in one embodiment, the light source 44 is an ultraviolet light source configured to emit light including a wavelength between approximately 200 and approximately 250 nm. If the light source 44 is configured to emit light having a broader band of wavelengths than what is absorbed by citrate (e.g., if a mercury vapor lamp is used as a light source), one or more filters 50 may be provided between the light source 44 and the light detector 46 to filter out any wavelengths falling outside of the target or ideal range of values. The nature of filters 50, if provided, may vary without departing from the scope of the present disclosure. For example, in one embodiment, the citrate detector 42 may be provided with a bandpass filter 50, a broad band filter 50a, and an ultraviolet bandpass filter 50b.

The light detector 46 is configured to receive the light emitted by the light source 44. Thus, if the light source 44 comprises an ultraviolet light source, the light detector 46 comprises an ultraviolet light detector. While the light detector 46 is active, it may send signals or data to the controller 30 of the system 10. Alternatively, the light detector 46 may send signals or data to an independent controller or processor. The controller or processor receives the signals or data and determines whether there is citrate present in the conduit 48 between the light source 44 and the light detector 46. If the controller or processor determines that there is citrate present in the conduit 48, it may generate an "error" output to alert the operator. This may be in any of a number of forms, such as an audible siren or alert, a message displayed on a video screen, or a combination of alerts. The output generated by the controller or processor may additionally serve to halt the operation of the system 10 (or at least selected components thereof) to prevent the infusion of sodium citrate to the blood source.

The manner in which the controller or processor determines the presence of citrate may be achieved in any of a variety of ways. In one embodiment, the controller or processor may determine or be provided with a baseline value that represents the amount of light received by the light detector 46 in the absence of citrate in the conduit 48. This determination of a baseline value may be carried out prior to fluid flow through the conduit 48. It may be advantageous for the controller or processor to determine a new baseline value for each procedure in the event that the amount of light emitted by the light source 44 decreases over time. The baseline value is compared to the signal(s) or data transmitted from the light detector 46 to the controller or processor while fluid is flowing through the conduit 48. If the signal(s) or data received by the controller or processor during fluid flow through the conduit 48 is equal to the baseline value, it is indicative of replacement fluid flowing through the conduit 48 (i.e., the absence of sodium citrate). In this case, the controller or processor may either generate no output or generate an "all clear" output that alerts the operator that the replacement fluid container has been properly connected to the disposable set 14. In one embodiment, such an output may be a prerequisite to the reinfusion of fluid to the blood source or other operations of the system 10. Alternatively, rather than requiring the signal(s) or data received by the controller or processor to be equal to the baseline value, it may be sufficient if the signal(s) or data is substantially or at least generally equal to the baseline value. For example, a preselected variation from the baseline value (e.g., a 5% deviation) may be allowed by the controller or processor when generating an "all clear" output.

On the other hand, if the signal(s) or data received by the controller or processor during fluid flow through the conduit 48 is not equal to the baseline value (on account of light being absorbed before reaching the light detector 46), it may be indicative of sodium citrate flowing through the conduit 48. The variation from the baseline value that triggers an "error" output may vary in different embodiments. For example, in some embodiments, an "error" output will not be generated by the controller or processor unless the received signal(s) or data is less than 50% of the baseline value, while in other embodiments an "error" output may be generated by a greater or lesser variation from the baseline value. In some embodiments, beyond simply determining whether citrate is present in the conduit 48, the controller or processor may determine the amount or concentration of citrate in the conduit 48. This may be achieved in any of a variety of ways, but may be based on the magnitude of the variation from the baseline value, which may correspond generally to the amount or concentration of citrate in the conduit 48 (i.e., a greater variation from the baseline value is indicative of a greater amount or concentration of citrate).

In addition to the aforementioned two outputs that may be generated by the controller or processor (i.e., an "error" output that is indicative of the presence of sodium citrate and an "all clear" output that is indicative of the absence of sodium citrate in the conduit 48), additional outputs may also be generated by the controller or processor. For example, the controller or processor may be programmed such that, if the signal(s) or data received by the controller or processor is not equal to the baseline value (or is greater than the preselected allowable variation from the baseline value), but less than the variation required to generate an "error" output, the controller or processor will generate a third output. This third output may be in the form of an alert for the system operator to check the containers 18 to ensure that the replacement fluid container is connected to the proper tubing or conduit 48. The controller may also be programmed to convert the variation data to a calculated concentration of citrate and display the concentration, if desired.

In addition to the light source 44 and light detector 46, the citrate detector 42 may include a variety of other components without departing from the scope of the present disclosure. For example, in the illustrated embodiment, the citrate detector 42 includes a heat-reducing shield or cover or member 52 adjacent to the light source 44, three filters 50-50b positioned between the light source 44 and the light detector 46, and a light-absorbing shield or cover or member 54 adjacent to the conduit 48. The heat-reducing member 52, if provided, controls heat exchange between the light source 44 and the system 10 or surrounding environment. Additionally, the wavelength of light emitted by some light sources may vary over time as the light source is in operation and heats up, in which case the heat-reducing member 52 may comprise a fan or heat sink or the like to help maintain the light source 44 at a lower temperature during use. In practice, it may be preferable for the light source 44 to be operated for only a short time during each processing cycle (e.g., only long enough to determine the content of the fluid in the conduit 48), rather than being operable during the entire cycle to reduce the risk of heat affecting the performance of the light source 44.

The filters 50-50b, if provided, condition the light passing from the light source 44 to the light detector 46 (e.g., by filtering out certain wavelengths), as described above in greater detail. The light-absorbing member 54, if provided, prevents extraneous light from the environment from being transmitted to the light detector 46 and may prevent the release of light from the light source 44 into the surrounding environment. For example, if the light source 44 is an ultraviolet light source, it may be advantageous for the light-absorbing member 54 to absorb ultraviolet light to prevent ultraviolet environmental light from reaching the light detector 46, which could affect the performance of the citrate detector 42.

The citrate detector 42 may be employed alone or in combination with a flow detector 56 (Fig. 6). If provided, the flow detector 56 determines whether there is fluid present in the conduit 48. The detectors 42 and 56 may be associated with each other (e.g., via the controller or processor), such that the operation of the citrate detector 42 is dependent upon the presence of fluid in the conduit 48 and will not be actuated until the flow detector 56 generates a signal or data to indicate that there is fluid flow through the conduit 48.

In one embodiment, the flow detector 56 includes a light source 58 (such as, but not limited to, one or more light-emitting diodes) and a light detector 60 (such as, but not limited to, a photodiode), with the light detector 60 being configured and oriented to receive light from the light source 58. The light source 58 and light detector 60 are spaced apart a sufficient distance to accommodate a portion of the replacement fluid conduit 48 therebetween. Preferably, the flow detector 56 is associated with a portion of the conduit 48 that is upstream of the citrate detector 42 to detect the presence of fluid flow through the conduit 48 before the fluid reaches the citrate detector 42.

The light source 58 emits a light having a wavelength that is absorbed by water or a water-based solution (e.g., saline or an anticoagulant containing sodium citrate), but not by the conduit 48. It has been found that infrared light is absorbed by water and water-based solutions, but at least partially transmitted by tubing or conduits manufactured from a variety of suitable materials. Accordingly, in one embodiment, the light source 58 is an infrared light source configured to emit light including a wavelength in the infrared range of values. If the light source 58 is configured to emit light having a broader band of wavelengths than what is absorbed by water and water-based solutions, one or more filters 62 may be provided between the light source 58 and the light detector 60 to filter out any wavelengths falling outside of the target or ideal range of values. In the illustrated embodiment, the flow detector 56 is shown with a bandpass filter 62, but other types of filters may also be employed without departing from the scope of the present disclosure.

The light detector 60 is configured to receive the light emitted by the light source 58. Thus, if the light source 58 comprises an infrared light source, the light detector 60 comprises an infrared light detector. While the light detector 60 is active, it may send signals or data to the controller 30 of the system 10 or to an independent controller or processor. It may be advantageous for the citrate detector 42 and the flow detector 56 to be associated with the same controller or processor. The controller or processor receives the signals or data and determines whether there is fluid flow in the conduit 48 between the light source 58 and the light detector 60. If the controller or processor determines that there is fluid present in the conduit 48, it may generate an output, such as a command to actuate the citrate detector 42.

The manner in which the controller or processor determines the presence of fluid in the conduit 48 may be achieved in any of a variety of ways. In one embodiment, the controller or processor may determine or be provided with a baseline value (which is a second baseline value that is different from the baseline value described above with regard to the citrate detector 42) that represents the amount of light received by the light detector 60 in the absence of fluid in the conduit 48. The baseline value is compared to the signal(s) or data transmitted from the light detector 60 to the controller or processor. If the signal(s) or data received by the controller or processor is equal to the baseline value, it is indicative of the absence of fluid in the conduit 48. In this case, the controller or processor may forego generating an output to actuate the citrate detector 42. Alternatively, rather than requiring the signal(s) or data received by the controller or processor to be equal to the baseline value, it may be sufficient if the signal(s) or data is substantially or at least generally equal to the baseline value. For example, a preselected variation from the baseline value (e.g., a 5% deviation) may be allowed by the controller or processor in determining that there is no fluid in the conduit 48.

On the other hand, if the signal(s) or data received by the controller or processor during fluid flow through the conduit 48 is not equal to the baseline value (on account of light being absorbed before reaching the light detector 60), it may be indicative of fluid flowing through the conduit 48. The variation from the baseline value that triggers a "power on" output or command (which triggers operation of the citrate detector 42) may vary in different embodiments. For example, in some embodiments, a "power on" output will not be generated by the controller or processor unless the received signal(s) or data is less than 90% of the baseline value, while in other embodiments a "power on" output or command may be generated by a greater or lesser variation from the baseline value. The controller may be programmed to convert the variation data to a calculated concentration or amount of fluid and display the concentration or amount, if desired.

In addition to the light source 58 and light detector 60, the flow detector 56 may include a variety of other components without departing from the scope of the present disclosure. For example, in the illustrated embodiment, the flow detector 56 includes the aforementioned filter 62 and a light-absorbing shield or cover or member 64 adjacent to the conduit 48. The light-absorbing member 64 provides the same function for the flow detector 56 as the light-absorbing member 54 provides for the citrate detector 42. Additionally, the light-absorbing members 54 and 64 may serve to prevent light from one detector from reaching the light detector of the other detector (e.g., preventing light from the light source 44 of the citrate detector 42 from reaching the light detector 60 of the flow detector 56). To further prevent light interference between the two detectors 42 and 56, it may be advantageous for them be spaced apart along the length of the conduit 48.

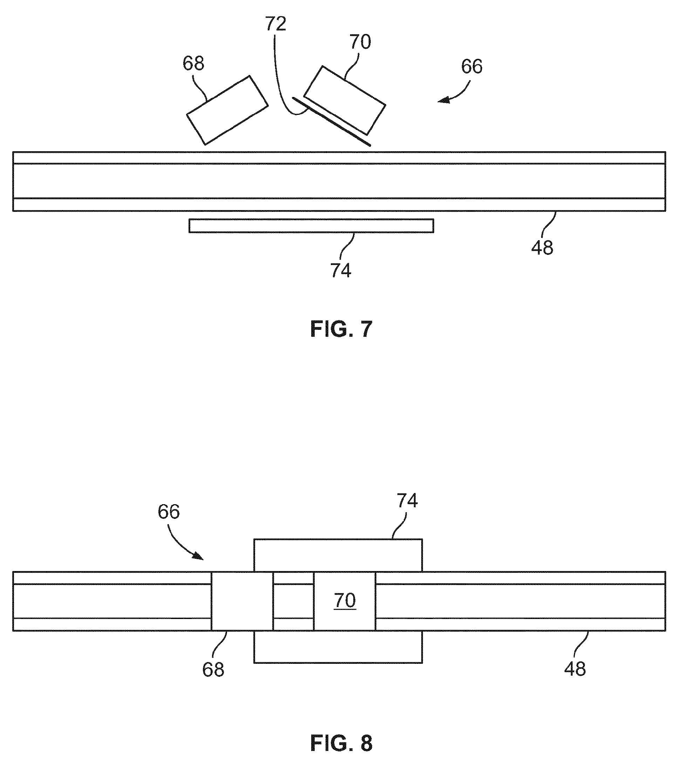

In some embodiments, a flow detector 56 incorporating an infrared light source may be capable of detecting the presence of citrate or a citrate solution. In such circumstances, the flow detector 56 may replace the citrate detector and perform the detection duties of both a flow detector and a citrate detector. For example, Figs. 7 and 8 show a citrate/flow detector 66 comprising an infrared light source 68 (e.g., a light-emitting diode or the like) and a light detector 70 (e.g., photodiode or the like) configured for receiving infrared light from the light source 68. While Figs. 7 and 8 illustrate the light source 68 and the light detector 70 as being physically separate components, they may be integrated into a common detector housing or frame or the like.

In contrast to the citrate detector 42 of Fig. 5, the light source 68 and light detector 70 are not positioned generally opposite each other, with the conduit 48 positioned therebetween. It has been found that infrared light reflected from a citrate-containing fluid (as well as other organic solutions) produces peak measurements that are not produced in infrared light reflected from a substantially citrate-free fluid (e.g., saline), such that, in the illustrated embodiment the light detector 70 is positioned and oriented so as to receive light from the light detector 70 that has been reflected from the conduit 48 and the fluid flowing therethrough. For example, in one embodiment, there is a peak for light having a wave number of approximately 1574 cm⁻¹ (or a wavelength of approximately 6,353.24 nm) that can be detected using infrared subtraction techniques. At such a wavelength, PVC is effectively transparent, meaning that the conduit 48 may be comprised of PVC (e.g., flexible PVC tubing), but may also be comprised of any other suitable material(s).

In addition to the light source 68 and the light detector 70, additional components may be included. For example, Figs. 7 and 8 illustrate a filter 72 and a reflector 74. The filter 72 is oriented to receive light prior to the light encountering the light detector 70, and more or fewer than one filter 72 may be provided. If provided, the filter 72 may be variously configured, but in one embodiment it comprises a bandpass filter that is configured to filter out wavelengths that are different from the target wavelength (e.g., a target wavelength of approximately 6,353.24 nm). As for the reflector 74, it is oriented and configured to concentrate reflected light on the light detector 70. The illustrated reflector 74 is positioned opposite the light source 68 and the light detector 70, with the conduit 48 positioned therebetween, but the reflector 74 (if provided) may be placed at other locations without departing from the scope of the present disclosure. The reflector 74 may be variously configured, but in one embodiment comprises a mirror or a similar surface with light-reflective properties. More or fewer than one reflector 74 may be provided, and if multiple reflectors 74 are provided, they may be similarly or differently configured without departing from the scope of the present disclosure.

It is also within the scope of the present disclosure for a flow detector to be used in combination with a separate citrate detector, in which case the citrate-detection functionality of the flow detector may be used as a back-up to the dedicated citrate detector, to decrease the risk of malfunction or failure. In other embodiments, the citrate detector itself may incorporate an infrared light source (as in Figs. 7 and 8) to detect the presence of citrate or a citrate solution. Furthermore, if a fluid being monitored by a detector having an infrared light source includes a plurality of components having specific characteristic infrared absorption properties, it may be possible to measure these values and determine the composition of the fluid based at least in part on the measurements.

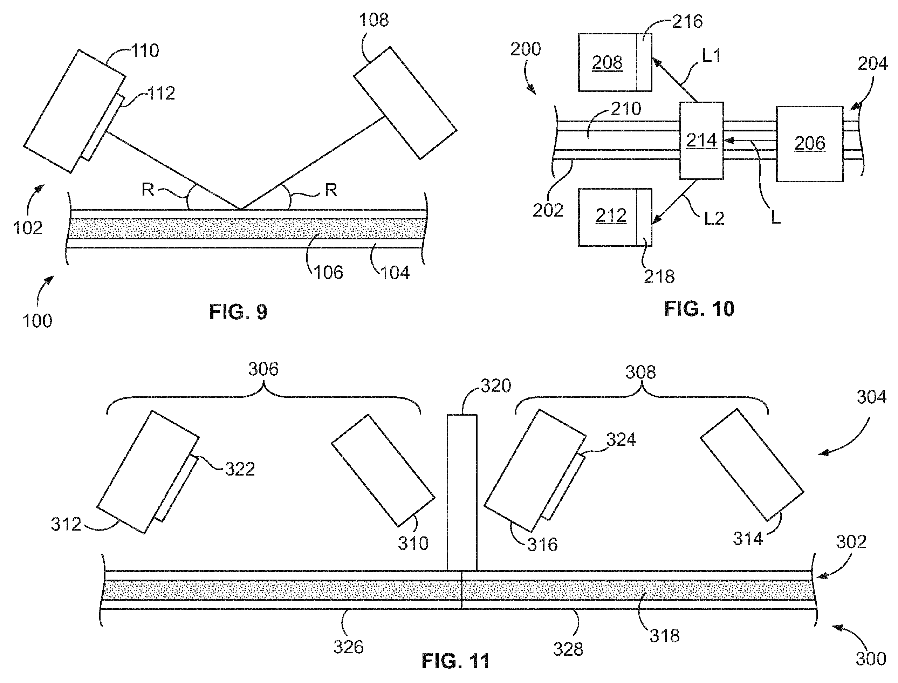

According to another aspect of the present disclosure, the level of light (e.g., infrared light) reflected by a fluid container (e.g., a fluid flow conduit) and a fluid or solution (which terms are used interchangeably herein) in the container may be quantified to identify the solution (or a constituent thereof) and/or to distinguish one solution from another solution. Fig. 9 illustrates an exemplary fluid identification system 100 for such fluid identification/differentiation. In the system 100 of Fig. 9, there is provided a fluid identification assembly 102 for use in combination with a container 104 in which is present a fluid or solution 106 to be analyzed. The container 104 may be part of a disposable processing set 14 or may be otherwise configured.

As for the fluid identification assembly 102, it may be provided generally in accordance with the foregoing description of a citrate detector, with a light source 108 (which may comprise one or more light-emitting diodes or be differently configured) and a light detector 110 (which may comprise a photodiode or be differently configured). The fluid identification assembly 102 may be part of a durable blood processing system 10 for monitoring intravenous fluids (e.g., organic fluids) or may be differently configured, including being provided as part of a system which handles non-bodily fluids. The light source 108 is angled or inclined toward the container 104, at an angle "R" between 0° and 90°, with the light detector 110 being configured and oriented to receive light from the light source 108 that has reflected by the container 104 and by the fluid 106 within the container 104. In one embodiment, the light source 108 and the light detector 110 are substantially aligned along the perimeter of the container 104, with each being angled or inclined toward the container 104 at an angle R of approximately 30°. In another embodiment, the light source 108 and the light detector 110 are substantially aligned along the perimeter of the container 104, with each being angled or inclined toward the container 104 at an angle R of approximately 45°. The optimal angle R may depend on any of a number of factors (e.g., the type of light used, the material composition of the container 104, and the nature of the fluid 106 to be analyzed), so the particular reflective angle R employed may vary without departing from the scope of the present disclosure.

The material composition of the container 104 depends upon the light emitted by the light source 108. As described above, the container 104 must be configured to reflect light from the light source 108, with at least a portion of that reflected light being received by the light detector 110. However, the container 104 must also transmit some of the light (preferably transmitting substantially all of the light or at least a substantial portion of the light), rather than reflecting and/or absorbing all of the light from the light source 108. By transmitting at least a portion of the light, the light may reach the fluid 106 within the container 104, where its interaction with the fluid 106 (namely, the amount of light absorbed by the fluid 106) is used to determine the identity of the fluid 106. By comparing the amount of light received by the light detector 110 from the light source 108 when the container 104 is empty to the amount of light received by the light detector 110 from the light source 108 when a fluid 106 is present within the container 104, the fluid identification assembly 102 may determine whether a particular fluid component is present in the fluid 106, as will be described in greater detail herein. By way of example, a container 104 formed of ethylene vinyl acetate or plasticized PVC may be suitable when analyzing the fluid 106 with light having a wavelength of approximately 6300 nm (if testing the fluid 106 for the presence of citrate, as described above) because substantially all of the light at that wavelength will pass through the container 104 and reach the fluid 106. Other container materials may be more appropriate when analyzing the fluid 106 using light having a different wavelength.

The wavelength at which the fluid 106 is analyzed depends upon the nature of the fluid 106 to be identified. For example, as described above in greater detail, light having a wavelength of approximately 6,353.24 nm or in the range of approximately 200 nm to approximately 250 nm may be used to identify the presence of citrate in the fluid 106, as such light is absorbed in large part by citrate. Other wavelengths may be used to identify other fluid components of the fluid 106 by analyzing the absorbance of different wavelengths of light by the target component and selecting a wavelength that is substantially absorbed by the target component. As described above, the material composition of the container 104 used in combination with the fluid identification assembly 102 should be selected to substantially transmit light of the wavelength of interest so that the majority of the light from the light source 108 reaching the container 104 passes through the container 104 and reaches the fluid 106.

If two fluids or fluid components have similar absorbance at a particular wavelength, then it may be advantageous to select another wavelength at which the two fluids or fluid components have different absorbance to distinguish the two. However, when attempting to distinguish between three or more fluids or fluid components, it may be advantageous to analyze the fluids or fluid components at two or more wavelengths. For example, four fluids may be provided, with each fluid absorbing light at first and second wavelengths λ1 and λ2 as shown in the below table:

**Table 1**

| Fluid | Absorbance (λ1) | Absorbance (λ2) |
|---|---|---|
| Fluid 1 | Low | Low |
| Fluid 2 | High | Low |
| Fluid 3 | Low | High |
| Fluid 4 | High | High |

It should be understood that the above table may represent a simplification of a more advanced approach, such as a table or database having precise data on the percentage of light having a particular wavelength that is absorbed by a variety of fluids and/or fluid components.

Using either one of the wavelengths may be sufficient to distinguish between two or more of the fluids (e.g., a low absorbance of light having wavelength λ1 may be used to distinguish Fluid 1 from Fluids 2 and 3), but both wavelengths must be employed to particularly identify each of the four fluids.

To allow a single light source 108 and a single light detector 110 to distinguish the four fluids in this situation, the fluid identification assembly 102 may be provided with a dual-band bandpass filter 112 positioned to cause at least a portion of the light from the light source 108 to pass through the dual-band bandpass filter 112 before reaching the light detector 110. The dual-band bandpass filter 112 of Fig. 9 is shown as intercepting the light that is reflected by the container 104 and fluid 106, but it is also within the scope of the present disclosure for the dual-band bandpass filter 112 to be positioned between the light source 108 and the container 104 to intercept the light before it is reflected by the container 104 and the fluid 106.

When a dual-band bandpass filter 112 is provided, the light source 108 may be provided as a broad wavelength source, with the dual-band bandpass filter 112 receiving the light and completely or at least substantially preventing all wavelengths other than λ1 and λ2 from passing through. Alternatively, rather than allowing only wavelengths λ1 and λ2 to pass through, the dual-band bandpass filter 112 may be configured to allow two ranges of wavelengths to pass through, with λ1 falling within the first range and λ2 falling within the second range. By such a configuration, the light detector 110 receives reflected light having wavelength λ1 and reflected light having λ2, with the magnitude of the light received depending on the fluid 106 within the container 104. If the amount of light of wavelength λ1 received by the light detector 110 is above a certain value, then it is indicative of the fluid 106 having a low absorbance of light having that wavelength, meaning that the fluid 106 may be Fluid 1 or Fluid 3 in the above example. On the other hand, if the amount of light of wavelength λ1 received by the light detector 110 is below a certain value, then it is indicative of the fluid 106 having a high absorbance of light having that wavelength, meaning that the fluid 106 may be Fluid 2 or Fluid 4 in the above example. Similarly, if the amount of light of wavelength λ2 received by the light detector 110 is above a certain value, then it is indicative of the fluid 106 having a low absorbance of light having that wavelength, meaning that the fluid 106 may be Fluid 1 or Fluid 2. Finally, if the amount of light of wavelength λ2 received by the light detector 110 is below a certain value, then it is indicative of the fluid 106 having a high absorbance of light having that wavelength, meaning that the fluid 106 may be Fluid 3 or Fluid 4. An amplifier and/or a noise-reduction device or filter may be associated with the light detector 110 to enhance and/or clarify the signal received by the light detector 110. Additionally or alternatively, a fixture may be incorporated into the fluid identification system 100, defining the path taken by the light from the light source 108 to the container 104 and to the light detector 110, with the portion of the fixture defining the path and facing the light being highly reflective (e.g., polished aluminum) to increase the amount of light that is ultimately directed to the light detector 110.

Considering the readings for the two wavelengths λ1 and λ2 allows for one of the four Fluids 1-4 to be particularly identified. The identification may be made by a controller or CPU or processor of the fluid indicator assembly 102 (which may correspond to the controller 30 of the system 10 or a separate device), which receives one or more signals from the light detector 110. The signals generated by the light detector 110 reflect the amount of light of each wavelength received by the light detector 110 and are, hence, indicative of the identity of the fluid 106. The controller may compare each signal to a baseline value or an expected value or some other value or values to ascertain whether a particular wavelength of light was highly/substantially absorbed by the fluid 106 or not. In one embodiment, use of the fluid identification system 100 may involve an initial calibration routine in which readings are taken of the amount of light of each wavelength received by the light detector 110 from the light source 108 when there is no fluid 106 in the container 104, thereby determining the effect of the container 104 on the light and allowing the controller to isolate the effect of the fluid 106 on the light.

When the fluid 106 has been identified, the fluid identification system 100 may take any of a number of actions. For example, it may generate an output indicative of the identity of the fluid 106 to cause the name of the fluid 106 to be displayed on a video screen. If it is determined that the fluid 106 should not be present in the container 104, then the fluid identification system 100 may generate an alarm and/or evacuate the fluid 106 from the container 104. For example, if the container 104 is a fluid flow conduit with the fluid 106 flowing therethrough, the flow of the fluid 106 may be reversed or diverted to a different destination. Other responses may be carried out without departing from the scope of the present disclosure.

Fig. 10 illustrates an alternative fluid identification system 200. Similar to the embodiment of Fig. 9, the fluid identification system 200 includes a container 202 and a fluid identification assembly 204 having a light source 206 and a light detector 208. As described above with respect to the embodiment of Fig. 9, the light source 206 is angled or inclined toward the container 202, with the light detector 208 being configured and oriented to receive light from the light source 206 that has reflected by the container 202 and by a fluid 210 within the container 202.

In addition to the light source 206 and the light detector 208, the fluid identification assembly 204 further includes a second light detector 212 (which may be substantially identical to the first light detector 208 or differently configured) and a beam splitter 214. The beam splitter 214 is positioned to receive at least a portion of the light "L" from the light source 206 that is reflected by the container 202 and the fluid 210 present within the container 202 and separate the light L into a first separated light beam "L1" and a second separated light beam "L2." The beam splitter 214 directs at least a portion of the first separated light beam L1 to the first light detector 208, while directing at least a portion of the second separated light beam L2 to the second light detector 212. One or both of the light detectors 208 and 212 may include an amplifier and/or a noise-reduction device or filter associated therewith to enhance and/or clarify the signal received by the light detector 208, 212. Additionally or alternatively, a fixture of the type described above with respect to the embodiment of Fig. 9 may be provided to define a light path from the light source 206 to the light detectors 208 and 212, thereby increasing the amount of reflected light ultimately received by the light detectors 208 and 212.

The beam splitter 214 may be variously configured without departing from the scope of the present disclosure. In one embodiment, the light source 206 may be a broad wavelength source, with the beam splitter 214 being configured to separate one wavelength of light (or range of wavelengths) from another wavelength of light (or range of wavelengths), and with the first separated light beam L1 having a first wavelength λ1 (or range of wavelengths) and the second separated light beam L2 having a second wavelength λ2 (or range of wavelengths). Such a beam splitter 214 may be provided as a diffraction grating, for example. In such an embodiment, the two light detectors 208 and 212 receive reflected light having different wavelengths, thereby allowing the fluid identification system 200 to determine whether first and second fluid components are present in the fluid 210 within the container 202 according to the principles described above with respect to the fluid identification system 100 of Fig. 9.

In another embodiment, the beam splitter 214 may be configured to separate a reflected light beam L into first and second separated light beams L1 and L2 having the same wavelength(s) as the initial light L. For example, such a beam splitter 214 may be provided as a series of mirrors or comparable reflective surfaces. In such an embodiment, each light detector 208, 212 may be provided with an associated bandpass filter, with the first bandpass filter 216 allowing only a first wavelength λ1 (or range of wavelengths) to pass through to the associated first light detector 208 and the second bandpass filter 218 allowing only a second wavelength λ2 (or range of wavelengths) to pass through to the associated second light detector 212. By providing such bandpass filters 216 and 218, the two light detectors 208 and 212 receive reflected light having different wavelengths, thereby allowing the fluid identification system 200 to determine whether first and second fluid components are present in the fluid 210 within the container 202 in accordance with the principles described above with respect to the fluid identification system 100 of Fig. 9.

Fig. 11 illustrates another embodiment of a fluid identification system 300. The embodiment of Fig. 11 includes a container 302 and a fluid identification assembly 304 having first and second modules 306 and 308. Preferably, the modules 306 and 308 are positioned adjacent to each other, such as shown in Fig. 11 with one module positioned directly upstream of the other module, but it is also within the scope of the present disclosure for the modules 306 and 308 to be farther apart. Each module 306, 308 may be provided generally in accordance with the foregoing description of the fluid identification assembly 102 of Fig. 9. In particular, the first module 306 includes a first light source 310 and a first light detector 312, while the second module 308 includes a second light source 314 and a second light detector 316. Each light source 310, 314 is angled or inclined toward the container 302, with the associated light detector 312, 316 being configured and oriented to receive light from the light source 310, 314 that has reflected by the container 302 and by the fluid 318 within the container 302. In the illustrated embodiment, a light barrier 320 is provided between the modules 306 and 308 to prevent the light from one light source 310, 314 from reaching the light detector 316, 312 of the other module 308, 306. One or both of the light detectors 312 and 316 may include an amplifier and/or a noise-reduction device or filter associated therewith to enhance and/or clarify the signal received by the light detector 312, 316. Additionally or alternatively, each module 306, 308 may be provided with a fixture of the type described above with respect to the embodiment of Fig. 9 to define a light path from a light source 310, 314 to the associated light detector 312, 316, thereby increasing the amount of reflected light ultimately received by the light detectors 312 and 316.

The light sources 310 and 314 may be configured to emit light having different wavelengths, such that the two light detectors 312 and 316 receive reflected light having different wavelengths, thereby allowing the fluid identification system 300 to determine whether first and second fluid components are present in the fluid 310 within the container 302 in accordance with the principles described above with respect to the fluid identification system 100 of Fig. 9. If one or both of the light sources 310, 314 are provided as broad wavelength sources instead of narrow wavelength sources, then the associated module 306, 308 may further include a band pass filter 322, 324 to filter out light not having the appropriate wavelength from the reflected light that is received by the associated light detector 312, 316. While single-band bandpass filters 322 and 322 are provided in one embodiment, it is also within the scope of the present disclosure for dual-band bandpass filters to be incorporated into the fluid identification system 300 of Fig. 11. Additionally, one or more beam splitters and additional light detectors (in accordance with the foregoing description of the embodiment of Fig. 10) may be incorporated into the fluid identification system 300 of Fig. 11. Furthermore, it is also within the scope of the present disclosure for the fluid identification system 300 to include more than two modules.

The embodiment of Fig. 11 is particularly advantageous when there is no single container material that substantially transmits light having all of the wavelengths of interest. For example, if wavelength λ1 is substantially transmitted through only container materials that substantially absorb wavelength λ2, then it may be necessary to provide a container 302 formed of different materials in the regions where light is being reflected by the container 302. In the illustrated embodiment, the container 302 is formed of two segments or sections 326 and 328, with the first segment 326 being configured and positioned to reflect light emitted by the first light source 310 and the second segment 328 being configured and positioned to reflect light emitted by the second light source 314. The first segment 326 is formed of a material that will substantially transmit light at a first wavelength being used to analyze the fluid 318 within the container 302, while the second segment 328 is formed of a different material (or the same materials in different amounts) that will substantially transmit light at a second wavelength being used to analyze the fluid 318 within the container 302. The segments 326 and 328 may be joined together by any suitable joint (e.g., a weld or a physical fastener) to define the container 302, with the nature of the joint depending on the material composition of the segments 326 and 328.

Aspects of the present subject matter described above may be beneficial alone or in combination with one or more other aspects. Without limiting the foregoing description, in accordance with one aspect of the subject matter herein, there is provided a fluid identification assembly for use in combination with a fluid container. The fluid identification assembly includes a light source configured to direct a light substantially transmitted by the fluid container to the fluid container for reflection by the fluid container and by a fluid present in the fluid container. A light detector is positioned to receive at least a portion of the reflected light, while a dual-band bandpass filter is positioned such that at least a portion of the light passes through the dual-band bandpass filter prior to being received by the light detector. The dual-band bandpass filter is configured to allow light having a first wavelength or a first range of wavelengths and a second wavelength or a second range of wavelengths to reach the light detector and to substantially prevent other wavelengths of light from reaching the light detector. The light detector is configured to, after receiving the reflected light, generate a signal indicative of the identity of the fluid present in the fluid container based, at least in part, on the amount of light that is received from the light source.

In accordance with another aspect which may be used or combined with the preceding aspect, there is provided an amplifier associated with the light detector.

In accordance with another aspect which may be used or combined with any of the preceding aspects, the dual-band bandpass filter is configured to allow the passage of two wavelengths of light that are substantially absorbed by different fluid components of an intravenous solution or the passage of two ranges of wavelengths of light including two wavelengths of light that are substantially absorbed by different fluid components of an intravenous solution.

In accordance with another aspect which may be used or combined with any of the preceding aspects, the fluid identification assembly is configured to differentiate between a fluid including no fluid component that substantially absorbs light having the first wavelength or a wavelength within the first range of wavelengths and no fluid component that substantially absorbs light having the second wavelength or a wavelength within the second range of wavelengths; a fluid including a fluid component that substantially absorbs light having the first wavelength or a wavelength within the first range of wavelengths, but no fluid component that substantially absorbs light having the second wavelength or a wavelength within the second range of wavelengths; a fluid including a fluid component that substantially absorbs light having the second wavelength or a wavelength within the second range of wavelengths, but no fluid component that substantially absorbs light having the first wavelength or a wavelength within the first range of wavelengths; and a fluid including a first fluid component that substantially absorbs light having the first wavelength or a wavelength within the first range of wavelengths and a second fluid component that substantially absorbs light having the second wavelength or a wavelength within the second range of wavelengths.

In accordance with another aspect which may be used or combined with any of the preceding aspects, the light source is configured to emit an infrared light.

In accordance with another aspect, there is provided a fluid identification assembly for use in combination with a fluid container. The fluid identification assembly includes a light source configured to direct a light substantially transmitted by the fluid container to the fluid container for reflection by the fluid container and by a fluid present in the fluid container. A beam splitter is positioned such that at least a portion of the light reflected by the fluid container and by the fluid present in the fluid container is intercepted by the beam splitter and separated into a first separated light beam and a second separated light beam. A first light detector is positioned to receive at least a portion of the first separated light beam, while a second light detector is positioned to receive at least a portion of the second separated light beam. The first light detector is configured to, after receiving its portion of the first separated light beam, generate a first signal indicative of the presence or absence of a first fluid component in the fluid present in the fluid container based, at least in part, on the amount of light that is received from the light source. The second light detector is configured to, after receiving its portion of the second separated light beam, generate a second signal indicative of the presence or absence of a second fluid component in the fluid present in the fluid container based, at least in part, on the amount of light that is received from the light source.

In accordance with another aspect which may be used or combined with the preceding aspect, the beam splitter comprises a diffraction grating.

In accordance with another aspect which may be used or combined with any of the preceding two aspects, there is provided a first bandpass filter, which is positioned such that at least a portion of the first separated light beam passes through the first bandpass filter prior to being received by the first light detector. There is also provided a second bandpass filter, which is positioned such that at least a portion of the second separated light beam passes through the second bandpass filter prior to being received by the second light detector. The first bandpass filter is configured to allow light having a first wavelength or a first range of wavelengths to reach the first light detector and to substantially prevent other wavelengths of light from reaching the first light detector. The second bandpass filter is configured to allow light having a second wavelength or a second range of wavelengths to reach the second light detector and to substantially prevent other wavelengths of light from reaching the second light detector. The first wavelength or a wavelength within the first range of wavelengths is selected to be substantially absorbed by the first fluid component, and the second wavelength or a wavelength within the second range of wavelengths is selected to be substantially absorbed by the second fluid component.

In accordance with another aspect which may be used or combined with any of the preceding three aspects, there is provided a first amplifier associated with the first light detector and a second amplifier associated with the second light detector.

In accordance with another aspect which may be used or combined with any of the preceding four aspects, the fluid identification assembly is configured to detect the presence or absence of the first and second fluid components of an intravenous solution.

In accordance with another aspect which may be used or combined with any of the preceding five aspects, the fluid identification assembly is configured to differentiate between a fluid including neither the first fluid component nor the second fluid component; a fluid including the first fluid component, but not the second fluid component; a fluid including the second fluid component, but not the first fluid component; and a fluid including the first and second fluid components.

In accordance with another aspect, there is provided a fluid identification assembly for use in combination with a fluid container. The fluid identification assembly includes a first module having a first light source and a first light detector. The first light source is configured to direct a first light substantially transmitted by the fluid container to the fluid container for reflection by the fluid container and by a fluid present in the fluid container, while the first light detector is positioned to receive at least a portion of the reflected first light. The fluid identification assembly also includes a second module having a second light source and a second light detector. The second light source is configured to direct a second light substantially transmitted by the fluid container to the fluid container for reflection by the fluid container and by a fluid present in the fluid container, while the second light detector is positioned to receive at least a portion of the reflected second light. The first light detector is configured to, after receiving its portion of the reflected first light, generate a first signal indicative of the presence or absence of a first fluid component in the fluid present in the fluid container based, at least in part, on the amount of light that is received from the first light source. The second light detector is configured to, after receiving its portion of the reflected second light, generate a second signal indicative of the presence or absence of a second fluid component in the fluid present in the fluid container based, at least in part, on the amount of light that is received from the second light source.

In accordance with another aspect which may be used or combined with the preceding aspect, there is provided a light barrier positioned between the first and second modules.

In accordance with another aspect which may be used or combined with any of the preceding two aspects, one of the modules is positioned adjacent to and upstream of the other module.

In accordance with another aspect which may be used or combined with any of the preceding three aspects, the first module includes a first bandpass filter positioned such that at least a portion of the first light passes through the first bandpass filter prior to being received by the first light detector. The second module includes a second bandpass filter positioned such that at least a portion of the second light passes through the second bandpass filter prior to being received by the second light detector. The first bandpass filter is configured to allow light having a first wavelength or a first range of wavelengths to reach the first light detector and to substantially prevent other wavelengths of light from reaching the first light detector, while the second bandpass filter is configured to allow light having a second wavelength or a second range of wavelengths to reach the second light detector and to substantially prevent other wavelengths of light from reaching the second light detector. The first wavelength or a wavelength within the first range of wavelengths is selected to be substantially absorbed by the first fluid component, while the second wavelength or a wavelength within the second range of wavelengths is selected to be substantially absorbed by the second fluid component.

In accordance with another aspect which may be used or combined with any of the preceding four aspects, there is provided a first amplifier associated with the first light detector and a second amplifier associated with the second light detector.

In accordance with another aspect which may be used or combined with any of the preceding five aspects, the fluid identification assembly is configured to detect the presence or absence of the first and second fluid components of an intravenous solution.

In accordance with another aspect which may be used or combined with any of the preceding six aspects, the fluid identification assembly is configured to differentiate between a fluid including neither the first fluid component nor the second fluid component; a fluid including the first fluid component, but not the second fluid component; a fluid including the second fluid component, but not the first fluid component; and a fluid including the first and second fluid components.

In accordance with another aspect which may be used or combined with any of the preceding thirteen aspects, at least one of the light sources is configured to emit an infrared light.

In accordance with another aspect, there is provided a fluid identification system which includes a fluid identification assembly according to any of the twelfth through eighteenth aspects. The fluid identification system further includes a fluid container having a first segment and a second segment formed of different materials or the same materials in different amounts. The first light is substantially transmitted by the first segment and directed to the fluid container by the first light source for reflection by the first segment and by a fluid present in the first segment. The second light is substantially transmitted by the second segment and directed to the fluid container by the second light source for reflection by the second segment and by a fluid present in the second segment.

In accordance with another aspect, there is provided a method of identifying a fluid in a fluid container. The method includes directing a light substantially transmitted by a fluid container to the fluid container for reflection by the fluid container and by a fluid present in the fluid container. Reflected light having a first wavelength or a first range of wavelengths and a second wavelength or a second range of wavelengths is detected, and the identity of the fluid present in the fluid container is determined based, at least in part, on the amount of reflected light that is detected.

In accordance with another aspect which may be used or combined with the preceding aspect, detecting reflected light includes detecting reflected light passing through a dual-band bandpass filter configured to allow light having the first wavelength or first range of wavelengths and the second wavelength or second range of wavelengths to pass through the dual-band bandpass filter and to substantially prevent other wavelengths of light from passing through the dual-band bandpass filter.

In accordance with another aspect which may be used or combined with the twenty-first aspect, the reflected light is split into a first separated light beam and a second separated light beam prior to detecting the reflected light.

In accordance with another aspect which may be used or combined with the twenty-first aspect, directing a light includes directing first and second lights substantially transmitted by the fluid container to the fluid container for reflection by the fluid container and by the fluid present in the fluid container. Detecting the reflected light includes detecting reflected first light having the first wavelength or first range of wavelengths using a first light detector and detecting reflected second light having the second wavelength or second range of wavelengths using a second light detector.

In accordance with another aspect which may be used or combined with any of the preceding four aspects, directing a light includes directing an infrared light to the fluid container for reflection by the fluid container and by the fluid present in the fluid container.

It will be understood that the embodiments described above are illustrative of some of the applications of the principles of the present subject matter. Numerous modifications may be made by those skilled in the art without departing from the scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A fluid identification assembly (102) for use in combination with a fluid container (104), the fluid identification assembly (102) comprising:
a light source (108) configured to direct a light substantially transmitted by the fluid container (104) to the fluid container (104) for reflection by the fluid container (104) and by a fluid (106) present in the fluid container (104);
a light detector (110) positioned to receive at least a portion of the reflected light; and
a dual-band bandpass filter (112) positioned such that at least a portion of the light passes through the dual-band bandpass filter (112) prior to being received by the light detector (110), wherein
the dual-band bandpass filter (112) is configured to allow light having a first wavelength or a first range of wavelengths and a second wavelength or a second range of wavelengths to reach the light detector (110) and to substantially prevent other wavelengths of light from reaching the light detector (110), and
the light detector (110) is configured to, after receiving said at least a portion of the reflected light, generate a signal indicative of the identity of the fluid (106) present in the fluid container (104) based, at least in part, on the amount of light received from the light source (108).

2. The fluid identification assembly (102) of claim 1, further comprising an amplifier associated with the light detector (110).

3. The fluid identification assembly (102) of any of the preceding claims, wherein the dual-band bandpass filter (112) is configured to allow the passage of two wavelengths of light that are substantially absorbed by different fluid components of an intravenous solution or the passage of two ranges of wavelengths of light including two wavelengths of light that are substantially absorbed by different fluid components of an intravenous solution.

4. The fluid identification assembly (102) of any of the preceding claims, configured to differentiate between
a fluid (106) including no fluid component that substantially absorbs light having the first wavelength or a wavelength within the first range of wavelengths and no fluid component that substantially absorbs light having the second wavelength or a wavelength within the second range of wavelengths,
a fluid (106) including a fluid component that substantially absorbs light having the first wavelength or a wavelength within the first range of wavelengths, but no fluid component that substantially absorbs light having the second wavelength or a wavelength within the second range of wavelengths,
a fluid (106) including a fluid component that substantially absorbs light having the second wavelength or a wavelength within the second range of wavelengths, but no fluid component that substantially absorbs light having the first wavelength or a wavelength within the first range of wavelengths, and
a fluid (106) including a first fluid component that substantially absorbs light having the first wavelength or a wavelength within the first range of wavelengths and a second fluid component that substantially absorbs light having the second wavelength or a wavelength within the second range of wavelengths.

5. The fluid identification assembly (102) of any of the preceding claims, wherein the light source (108) is configured to emit an infrared light.

6. The fluid identification assembly (102) of any of the preceding claims, wherein the light source (108) is configured to direct light to the fluid container (104) while the fluid (106) is flowing through the fluid container (104) and the signal generated by the light detector (110) is indicative of the identify of the fluid (106) flowing through the fluid container (104).

7. A fluid identification system (100) comprising the fluid identification assembly (102) of any of the preceding claims and a fluid circuit including the fluid container (104).

8. The fluid identification system (100) of claim 7, wherein the fluid circuit comprises an extracorporeal blood flow circuit.

9. The fluid identification system (100) of any of claims 7-8, wherein the fluid container (104) is formed of ethylene vinyl acetate or plasticized polyvinyl chloride.

10. A method for identifying a fluid (106, 210, 318) in a fluid container (104, 202, 302) comprising:
directing a light substantially transmitted by a fluid container (104, 202, 302) to the fluid container (104, 202, 302) for reflection by the fluid container (104, 202, 302) and by a fluid (106, 210, 318) present in the fluid container (104, 202, 302);
detecting reflected light having a first wavelength or a first range of wavelengths and a second wavelength or a second range of wavelengths; and
determining the identity of the fluid (106, 210, 318) present in the fluid container (104, 202, 302) based, at least in part, on the amount of reflected light detected.

11. The method of claim 10, wherein said detecting reflected light includes detecting reflected light passing through a dual-band bandpass filter (112) configured to allow light having said first wavelength or first range of wavelengths and said second wavelength or second range of wavelengths to pass through the dual-band bandpass filter (112) and to substantially prevent other wavelengths of light from passing through the dual-band bandpass filter (112).

12. The method of claim 10, further comprising splitting the reflected light into a first separated light beam (L1) and a second separated light beam (L2) prior to said detecting reflected light.

13. The method of claim 10, wherein
said directing a light includes directing first and second lights substantially transmitted by the fluid container (302) to the fluid container (302) for reflection by the fluid container (302) and by the fluid (318) present in the fluid container (302), and
said detecting reflected light includes detecting reflected first light having said first wavelength or first range of wavelengths using a first light detector (312) and detecting reflected second light having said second wavelength or second range of wavelengths using a second light detector (316).

14. The method of any of claims 10-13, wherein said directing a light includes directing an infrared light to the fluid container (104, 202, 302) for reflection by the fluid container (104, 202, 302) and by the fluid (106, 210, 318) present in the fluid container (104, 202, 302).

15. The method of any of claims 10-14, wherein said directing a light includes directing the light to the fluid container (104, 202, 302) while the fluid (106, 210, 318) is flowing through the fluid container (104, 202, 302).
